(19) Europäisches Patentamt
European Patent Office
Office européen des brevets

(11) **EP 3 766 472 A1**

(12) **EUROPEAN PATENT APPLICATION**
published in accordance with Art. 153(4) EPC

(43) Date of publication:
**20.01.2021 Bulletin 2021/03**

(21) Application number: **19768323.8**

(22) Date of filing: **12.03.2019**

(51) Int Cl.:
**A61J 1/05** (2006.01)

(86) International application number:
**PCT/JP2019/009883**

(87) International publication number:
**WO 2019/176902 (19.09.2019 Gazette 2019/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.03.2018 JP 2018045884**

(71) Applicant: Santen Pharmaceutical Co., Ltd.
**Kita-ku**
**Osaka-shi**
**Osaka 530-8552 (JP)**

(72) Inventors:
• **YAMAGATA, Tomoko**
  **Osaka-shi, Osaka 530-8552 (JP)**
• **OSHITA, Yoshihiro**
  **Osaka-shi, Osaka 530-8552 (JP)**
• **KUSAOKE, Daiki**
  **Inukami-gun, Shiga 522-0314 (JP)**

(74) Representative: **Müller-Boré & Partner**
  **Patentanwälte PartG mbB**
  **Friedenheimer Brücke 21**
  **80639 München (DE)**

(54) **INSTILLATION ASSISTANCE TOOL**

(57) Provided is an ocular instillation assisting tool which makes it possible to accurately administer eye drops to an eye of a target of ocular instillation, with a simple structure. According to an ocular instillation assisting tool (1) of the present invention, an ocular guide (20) is formed so that, in a state where a target of ocular instillation lies on his or her back and the ocular guide (20) abuts on a periphery of an eye, a drop of eye drops contained in an eye drop container (30) drops at a position (A) which is shifted from the center (X) of a surface of the eye toward and an upper eyelid by a predetermined distance.

FIG. 1

**Description**

Technical Field

The present invention relates an ocular instillation assisting tool for assisting ocular instillation.

Background Art

Generally, ocular instillation with respect to a patient is performed by dropping eye drops into an eye of the patient with the face of the patient up. However, it is difficult to accurately administer the eye drops to the eye of the patient. Under the circumstances, for example, Patent Literature 1 discloses a guide for an eye drop dispenser bottle, the guide including a positioning member that causes an eyelid to remain open and thereby allowing eye drops to be accurately administered to an eye of a patient.

Citation List

[Patent Literature]

[Patent Literature 1]
Published Japanese Translation of PCT International Application Tokuhyo No. 2016-508054 (published on March 17, 2016)

Summary of Invention

Technical Problem

However, the guide for an eye drop dispenser bottle disclosed in Patent Literature 1 includes, as an essential constituent element, hinges adapted to rotate the positioning member between an operational position (ocular instillation position) and a stowed position. The guide including such hinges has a problem that, in a case where a frequency of use of the guide is high, a trouble, such as breakage of the hinges, is likely to occur and, consequently, the guide has decreased reliability as a guide. Moreover, since the guide itself has a complicated structure, there is a problem that a cost for production of the guide is high. Furthermore, unless the eye drops are accurately instilled, an effect of an active ingredient of the eye drops is not sufficiently exhibited. In this case, it takes a long period of time before an eye disease is cured, and there is a possibility of a situation that the eye disease is not cured. As a result, problems, such as an increase in mental burden on the patient and a significant increase in medical expense, also arise.
An object of an aspect of the present invention is to realize an ocular instillation assisting tool which, with a simple structure, allows eye drops to be accurately administered to an eye of a target of ocular instillation.

Solution to Problem

In order to attain the above object, an ocular instillation assisting tool in accordance with an aspect of the present invention is an ocular instillation assisting tool to which an eye drop container containing eye drops is detachably attached, the ocular instillation assisting tool including a positioning member which is caused to abut on a periphery of an eye of a target of ocular instillation so that a position on the eye at which position a drop of the eye drops drops is determined, the positioning member being formed so that, in a state where the target of the ocular instillation lies on his or her back and the positioning member abuts on the periphery of the eye, the drop of the eye drops contained in the eye drop container drops at a position which is shifted from a center of a surface of the eye toward an upper eyelid by a predetermined distance.
According to the above configuration, the positioning member is formed so that, in a state where the target of the ocular instillation lies on his or her back and the positioning member abuts on the periphery of the eye, the drop of the eye drops contained in the eye drop container drops at the position which is shifted from the center of the surface of the eye toward the upper eyelid by the predetermined distance. With this configuration, in a case where the target of the ocular instillation lies on his or her back, the position at which the eye drops drop is shifted from the center of the eye toward the upper eyelid. In a case where the target of the ocular instillation gradually gets up from a supine posture, the position at which the eye drops drop also gradually moves toward a lower eyelid. Therefore, in either of a case where a posture in which the ocular instillation is performed is a sitting posture and a case where the posture in which the ocular instillation is performed is a standing posture, it is possible to appropriately drop the eye drops into the eye.
Besides, it is possible to appropriately administer the eye drops to the eye of the target of the ocular instillation, by simply forming the positioning member so that, in a state where the target of the ocular instillation lies on his or her back and the positioning member abuts on the periphery of the eye, the drop of the eye drops contained in the eye drop container drops at the position which is shifted from the center of the surface of the eye toward the upper eyelid by the predetermined distance. Furthermore, since it is possible to accurately instill the eye drops, an effect of an active ingredient of the eye drops is sufficiently exhibited. As a result, it is expected that (i) an eye disease is cured in a brief period of time, (ii) a mental burden on a patient is reduced, and (iii) a medical expense is significantly suppressed.

The ocular instillation assisting tool is preferably arranged such that a part of the positioning member which part faces a lower eyelid in a case where the positioning member is caused to abut on the periphery of the eye is cut out.

According to the above configuration, the part of the positioning member which part faces the lower eyelid in a case where the positioning member is caused to abut on the periphery of the eye is cut out. This makes it possible to put a finger in such a cutout and pull the lower eyelid downward. It is therefore possible to more accurately administer the eye drops to the eye.

Moreover, by putting the finger in the cutout, it is possible to prevent the positioning member from unexpectedly rotating. This makes it easy to cause the positioning member to abut on a desired position.

The ocular instillation assisting tool is preferably arranged such that parts of the positioning member which parts respectively abut on an outer corner and an inner corner of the eye in a case where the positioning member is caused to abut on the periphery of the eye are protruded toward the face of the target of the ocular instillation, as compared with the other parts.

According to the above configuration, the parts of the positioning member, which parts respectively abut on the outer corner and the inner corner of the eye in a case where the positioning member is caused to abut on the periphery of the eye, are protruded toward the face of the target of the ocular instillation, as compared with the other parts. This makes it possible to easily cause the positioning member to abut on the periphery of the eye in line with the outer corner and the inner corner of the eye, the outer corner and the inner corner being recessed as compared with the other parts. It is therefore easy to cause the positioning member to abut on an appropriate position.

The ocular instillation assisting tool is preferably arranged such so as to further include an attachment member which is connected to the positioning member and to which the eye drop container is detachably attached, the attachment member having (i) a first opening through which a container body of the eye drop container is attached to and detached from the attachment member and (ii) at a position which faces the first opening, a second opening through which the container body of the eye drop container that is attached to the attachment member is partially exposed.

According to the above configuration, it is possible to push the container body of the eye drop container which container body is exposed through two openings in the attachment member in a state where the eye drop container is attached to the attachment member integrally formed with the positioning member.

Advantageous Effects of Invention

According to an aspect of the present invention, it is possible to, with a simple structure, accurately administer eye drops to an eye of a target of ocular instillation. Furthermore, since it is possible to accurately instill the eye drops, an effect of an active ingredient of the eye drops is sufficiently exhibited. As a result, it is expected that (i) an eye disease is cured in a brief period of time, (ii) a mental burden on a patient is reduced, and (iii) a medical expense is significantly suppressed.

Brief Description of Drawings

Fig. 1 is a schematic perspective view of an ocular instillation assisting tool in accordance with Embodiment 1 of the present invention.
Fig. 2 is a schematic perspective view of the ocular instillation assisting tool illustrated in Fig. 1, as viewed from a different angle.
Fig. 3 is a drawing illustrating how to perform ocular instillation with use of the ocular instillation assisting tool illustrated in Fig. 1.
Fig. 4 is a drawing illustrating a position of a nozzle for ocular instillation which nozzle is provided to an eye drop container attached to the ocular instillation assisting tool illustrated in Fig. 1.
Fig. 5 is a drawing illustrating conditions under which an ocular instillation experiment is carried out with use of the ocular instillation assisting tool illustrated in Fig. 1.
Fig. 6 is a drawing illustrating a success rate of the ocular instillation which success rate depends on an ocular instillation distance and an ocular instillation angle that are employed in the ocular instillation experiment carried out with use of the ocular instillation assisting tool illustrated in Fig. 1.

Fig. 7 is a drawing showing an example of the ocular instillation experiment.
Fig. 8 is a drawing showing another example of the ocular instillation experiment.
Fig. 9 is a drawing showing still another example of the ocular instillation experiment.
Fig. 10 is a schematic block diagram illustrating a configuration of a medication management system.

Description of Embodiments

Embodiment 1

The following description will discuss Embodiment 1 of the present invention in detail.

<Outline of ocular instillation assisting tool>

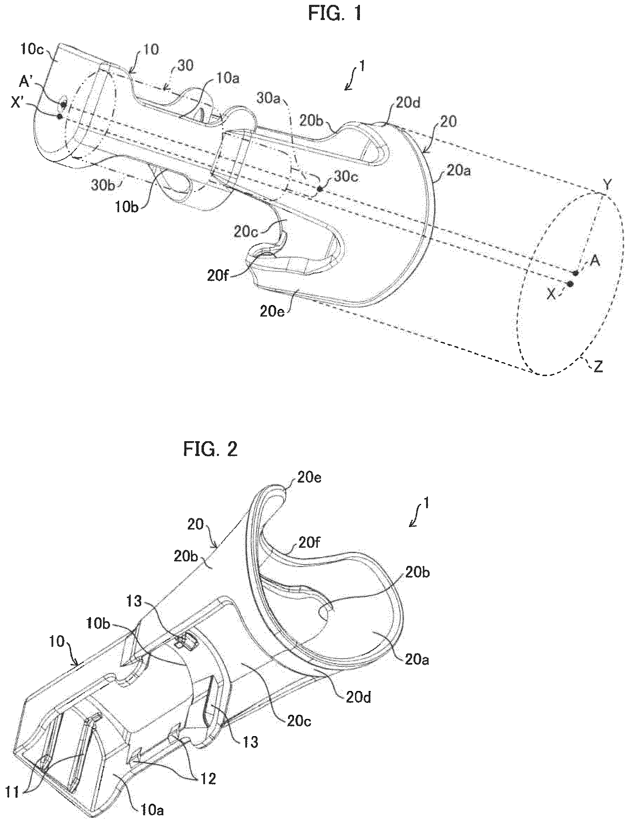

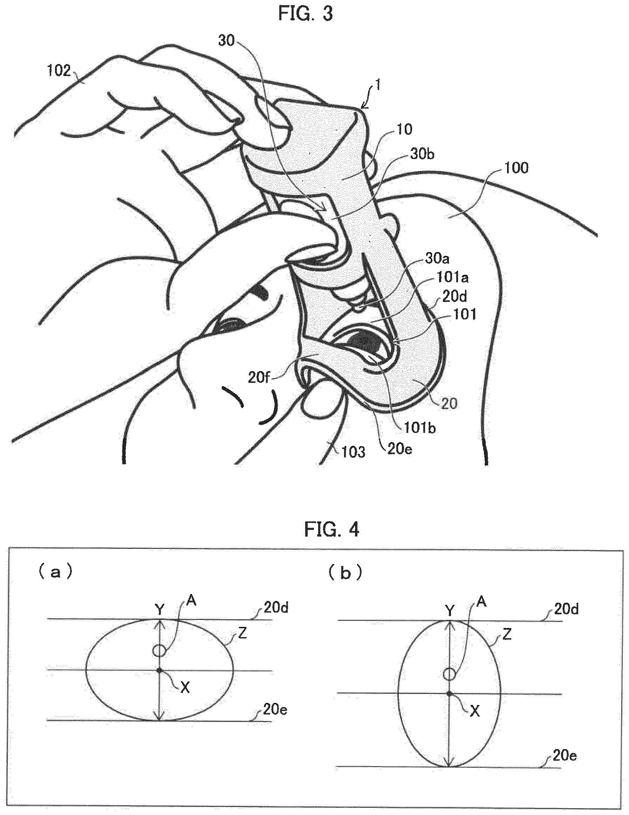

An outline of an ocular instillation assisting tool will be described below with reference to Fig. 3. Fig. 3 illustrates an example of how to use an ocular instillation assisting tool 1 in accordance with Embodiment 1 of the present invention.
As illustrated in Fig. 3, the ocular instillation assisting tool 1 is an assisting tool which assists administration of eye drops to a patient 100 (user, target of ocular instillation, target of administration). The ocular instillation assisting tool 1 includes an assisting tool body (attachment member) 10 and an ocular guide (positioning member) 20. Note that Embodiment 1 will be described based on an example in which the patient 100, by himself or herself, instills the eye drops from an eye drop container (container body) 30 with use of the ocular instillation assisting tool 1. That is, the patient 100 is a user of the ocular instillation assisting tool 1 and is also a target of the administration of the eye drops. Note that the user of the ocular instillation assisting tool 1 and the target of the administration may be different persons.
A specific structure of the ocular instillation assisting tool 1 will be described below with reference to Figs. 1 and 2. Fig. 1 is a schematic perspective view of the ocular instillation assisting tool 1 in accordance with Embodiment 1. Fig. 2 is a schematic perspective view of the ocular instillation assisting tool 1 illustrated in Fig. 1, as viewed from a different angle. Note that the eye drop container 30 which is used in a state of being attached to the ocular instillation assisting tool 1 is constituted by (i) an eye drop expelling part 30a from which the eye drops are expelled and (ii) an eye drop containing part 30b which contains the eye drops.

<Assisting tool body 10>

As illustrated in Fig. 1, the assisting tool body 10 substantially has a cylindrical shape, and is an attachment member to which the eye drop container 30, which contains the eye drops, is detachably attached. The assisting tool body 10 has, in its circumference, (i) a first opening 10a through which the eye drop container 30 is attached to and detached from the assisting tool body 10 and (ii) at a position which faces the first opening 10a, a second opening 10b which is smaller than the first opening 10a. With this configuration, in a case where the eye drop containing part 30b of the eye drop container 30 is attached to the assisting tool body 10 so that the eye drop expelling part 30a of the eye drop container 30 is directed to the ocular guide 20, the eye drop containing part 30b is partially exposed through the first opening 10a and the second opening 10b. By the user directly pushing, with his or her fingers, the eye drop containing part 30b of the eye drop container 30, which eye drop containing part 30b is partially exposed from the assisting tool body 10, it is possible to expel the eye drops contained in the eye drop containing part 30b from the eye drop expelling part 30a.
A side surface of the assisting tool body 10, in which side surface the first opening 10a and the second opening 10b are not provided, is cut out. This causes an exposed portion of the eye drop containing part 30b of the eye drop container 30 attached to the assisting tool body 10 to be large in area, thereby allowing the user to more easily push the eye drop containing part 30b.
The assisting tool body 10 further has, on its inner surface, first ribs 11, second ribs 12, and third ribs 13. In a case where the eye drop container 30 is attached to the assisting tool body 10, the first ribs 11 are brought into contact with a bottom surface of the eye drop containing part 30b, the second ribs 12 are brought into contact with side surfaces of the eye drop containing part 30b, and the third ribs 13 are brought into contact with side portions of an upper surface of the eye drop containing part 30b. The eye drop containing part 30b of the eye drop container 30 attached to the assisting tool body 10 is securely held by the first ribs 11, the second ribs 12, and the third ribs 13. Note, however, that the eye drop containing part 30b and the assisting tool body 10 each need to have elasticity. That is, the eye drop containing part 30b is held in the assisting tool body 10 by utilization of the elasticity of each of the eye drop containing part 30b and the assisting tool body 10.
Note that, by (i) causing an end part of the assisting tool body 10, which end part is opposite to the ocular guide 20, to have a planar shape and (ii) placing, on a planar surface such as a desk, the assisting tool body 10 with the end part down, it is possible to cause the ocular instillation assisting tool 1 to stand on its own.

<Ocular guide 20>

The ocular guide 20 is a member which is connected to the assisting tool body 10 and which is brought into contact with a periphery of an eye 101 of the patient 1 as illustrated in Fig. 3. The ocular guide 20 and the assisting tool body 10 may be integrally formed or may be alternatively formed separately.

As illustrated in Fig. 1, the ocular guide 20 has (i) a first opening 20a whose edge is brought into direct contact with the periphery of the eye 101 of the patient 1, (ii) a second opening 20b which is communicated with the first opening 10a of the assisting tool body 10, and (iii) a third opening 20c which is provided so as to be adjacent to the second opening 10b of the assisting tool body 10.

The second opening 20b and the third opening 20c of the ocular guide 20 are provided so that, in a case where the eye drop container 30 is attached to the assisting tool body 10, the eye drop expelling part 30a of the eye drop container 30 is exposed therethrough. This allows the user to, in a state where the eye drop container 30 is attached to the assisting tool body 10, (i) put his or her fingers in the second opening 20b and the third opening 20c and (ii) put a cap (not illustrated) on or remove the cap from the eye drop expelling part 30a of the eye drop container 30.

In a case where the ocular instillation assisting tool 1 is used, an upper edge 20d of the first opening 20a of the ocular guide 20, which upper edge 20d is located on a second-opening-20b side, is caused to abut on an upper-eyelid-lOla side of the eye 101 of the patient 100 as illustrated in Fig. 3. Then, a lower edge 20e of the first opening 20a, which lower edge 20e is located on a third-opening-20c side, is caused to abut on a lower-eyelid-101b side of the eye 101 of the patient 100.

The lower edge 20e of the first opening 20a has a curved part 20f which is curved toward the third opening 20c. The curved part 20f has a size that allows the patient 100 to put a finger of his or her left hand 103 therein in a case where the patient 100 operates the ocular instillation assisting tool 1 with his or her right hand 102. This makes it possible to put the finger in the curved part 20f and lower a lower eyelid 101b in a state where the ocular guide 20 is in contact with the periphery of the eye 101. Note that Fig. 3 illustrates an example in which the patient 100 uses the ocular instillation assisting tool 1 while operating the ocular instillation assisting tool 1 with the right hand 102 and putting the finger of the left hand 103 in the curved part 20f of the ocular guide 20, but the patient 100 may alternatively use the ocular instillation assisting tool 1 while operating the ocular instillation assisting tool 1 with the left hand 103 and putting a finger of the right hand 102 in the curved part 20f of the ocular guide 20. Thus, the ocular instillation assisting tool 1 may have a symmetric structure. Further, the ocular instillation assisting tool 1 may have such an asymmetric structure that lowering the lower eyelid 101b is assigned to one of the right and left hands and pushing the eye drop containing part 30b is assigned to the other of the right and left hands. That is, the ocular instillation assisting tool 1 may have a right-handed structure or a left-handed structure.

In this manner, providing the curved part 20f to the ocular guide 20 makes it possible to guide the ocular guide 20 to a predetermined position by putting the finger in the curved part 20f in a case where the ocular guide 20 is caused to abut on the periphery of the eye 101. That is, it is possible to appropriately position the ocular guide 20.

Moreover, in order that the ocular guide 20 is appropriately positioned, out of parts of the ocular guide 20 which parts are caused to abut on the periphery of the eye 101, parts of the ocular guide 20 which parts respectively correspond to an outer corner and an inner corner of the eye 101 are protruded, as compared with the other parts. With this configuration, in a case where the ocular guide 20 is caused to abut on the periphery of the eye 101, each of such protrusions serves as a guide and allows the ocular guide 20 to be guided to the predetermined position.

Note that, according to the ocular instillation assisting tool 1, the assisting tool body 10 and the ocular guide 20 are each made of polypropylene, which is an elastic material.

However, a material of the ocular instillation assisting tool 1 is not limited to polypropylene. The material of the ocular instillation assisting tool 1 may be any material, provided that the any material has water resistance, chemical resistance, and elasticity.

Note also that the eye drop container 30 used in the ocular instillation assisting tool 1 is preferably made of a material which has elasticity, excellent water resistance, and excellent chemical resistance, similarly to the ocular instillation assisting tool 1.

<Positioning by ocular guide 20>

Positioning by the ocular guide 20 of the ocular instillation assisting tool 1 will be described below with reference to Figs. 1, 3, and 4.

The center of a virtual region (first region) (dotted circle in Fig. 1; hereinafter, referred to as a virtual circle Z) is referred to as X, the virtual region being obtained by, in a state where the eye drop container 30 is attached to the assisting tool body 10 of the ocular instillation assisting tool 1 and the ocular guide 20 abuts on the periphery of the eye of the target of the ocular instillation, (i) projecting, from an assisting-tool-body-10 side toward a plane horizontal to a floor surface, the parts of the ocular guide 20 which parts abut on the periphery of the eye and (ii) connecting the parts with each other (see Fig. 1). Further, in the virtual circle Z, a point on which the upper edge 20d of the ocular guide 20 is projected is referred to Y. Here, a distance from the center X to the point Y corresponds to a radius of the virtual circle Z. That is, the distance from the center X to the point Y corresponds to a distance from the center of the ocular guide 20 to the upper edge 20d of the ocular guide 20, which the upper edge 20d is located on the upper-eyelid-101a side, in a case where the ocular guide 20 is caused to abut on the periphery of the eye 101 of the patient, as illustrated in Fig. 3. The distance from the center X of the virtual circle Z to the point Y is preferably 10 mm to 30 mm, more preferably 12.5 mm to 25 mm, and particularly preferably 15 mm to 20 mm. Further, a reached point which an expelling hole 30c of the eye drop expelling part 30a of the eye drop container 30 reaches if the expelling hole 30c is caused to linearly extend toward the virtual circle Z so as to reach the virtual circle Z is referred to as A. Here, the ocular guide 20 is designed so that the reached point A is shifted from the center X toward the upper edge 20d (the upper-eyelid-101a side of the eye 101) of the ocular guide 20 by a predetermined distance. Note that the predetermined distance corresponds to a distance obtained by subtracting a distance between the point Y and the reached point A from a distance between the point Y and the center X, the point Y being a point at which a perpendicular line extending from the upper edge 20d of the ocular guide 20 toward the virtual circle Z intersects the virtual circle Z, the reached point A being a point at which a perpendicular line extending from the expelling hole 30c toward the virtual circle Z intersects the virtual circle Z.

Specifically, in a case where the ocular guide 20 is caused to abut on the periphery of the eye 101 of the patient 100, the ocular guide 20 abuts on at least a vicinity of an outer side of the outer corner of the eye 101 and a vicinity of an outer side of the inner corner of the eye 101 (see Fig. 3). In this case, the virtual circle Z, which is formed on a plane of projection on which plane the ocular guide 20 is projected by being projected in a direction in which the eye drops are expelled, includes parts corresponding to the parts of the ocular guide 20 which parts abut on the vicinity of the outer side of the outer corner of the eye 101 and on the vicinity of the outer side of the inner corner of the eye 101. The ocular guide 20 is formed so that the expelling hole 30c, which is provided in the eye drop expelling part 30a of the eye drop container 30 attached to the assisting tool body 10 and through which the eye drops are expelled, is located at a position corresponding to a position which is shifted from the center X of the virtual circle Z, which is formed on the plane of projection, toward an upper eyelid 101a by the predetermined distance.

In other words, the ocular guide 20 only needs to be formed so that, in a state where the target of the ocular instillation lies on his or her back and the ocular guide 20 abuts on the periphery of the eye 101, a drop of the eye drops contained in the eye drop container 30 drops at a position which is shifted from the center of a surface (pupil 101c) of the eye 101 toward the upper eyelid 101a by the predetermined distance.

Note that a relationship between the reached point A and the center X in the virtual circle Z can be also defined as follows. That is, a state is assumed where the patient 100 lies on his or her back and the ocular guide 20 abuts on the periphery of the eye 101. Further, it is assumed that, in such a state, a part of the ocular guide 20 which part abuts on the upper eyelid is located on an upper side (Y direction in Fig. 1). Moreover, a point obtained by projecting, on a bottom surface 10c of the assisting tool body 10, the reached point A in the virtual circle Z, which is obtained by projecting the ocular guide 20 on the floor surface, is referred to as a projected point (first point) A'. Besides, a point obtained by projecting, on the bottom surface 10c, the center X of the virtual circle Z is referred to as a projected point (second point) X'. In this case, the ocular instillation assisting tool 1 is designed so that the projected point A' is shifted from the projected point X' toward the upper side by the predetermined distance.

The predetermined distance is preferably 2 mm to 4 mm, and particularly preferably 3 mm. In a case where the predetermined distance is shorter than 2 mm or longer than 4 mm, a problem that a probability of success of the ocular instillation is decreased arises.

In a case where the ocular instillation is performed with use of the ocular instillation assisting tool 1 configured as described above, it is possible to drop the eye drops, which are expelled from the eye drop container 30, on the upper-eyelid-101a side of the center of the eye. In a state where the patient 100 looks up in a sitting or standing posture, a position at which the eye drops drop is shifted toward the lower eyelid 101b. However, since the ocular guide 20 is designed so that the eye drops, which are expelled from the eye drop container 30, drop on the upper-eyelid-101a side of the center of the eye, it is possible to drop the eye drops so that the eye drops do not drop on the lower-eyelid-101b of the eye 101. Therefore, as compared with a case where an ocular instillation guide is designed so that the position at which the eye drops drop is superposed on the center of the eye 101, it is possible to accurately drop the eye drops even in a case where the surface targeted for the ocular instillation is tilted.

Besides, it is possible to accurately administer the eye drops to the eye of the patient, by simply forming the ocular guide 20 so that, in a state where the target of the ocular instillation lies on his or her back and the ocular guide 20, that is, the positioning member abuts on the periphery of the eye, the drop of the eye drops contained in the eye drop container 30 drops at the position which is shifted from the center of the surface of the eye 101 toward the upper eyelid 101a by the predetermined distance.

The expelling hole 30c, through which the eye drops are expelled, only needs to be located at the position corresponding to the position which is shifted from the center X of the virtual circle Z toward the point Y on the upper-edge-20d side of the ocular guide 20 by the predetermined distance. Therefore, the expelling hole 30c may have a shape of an ellipse which is longer in a lateral direction as illustrated in (a) of Fig. 4 or may alternatively have a shape of an ellipse which is longer in a longitudinal direction as illustrated in (b) of Fig. 4 or may alternatively have any other shape, regardless of an outer shape of the ocular guide 20. Furthermore, the virtual circle Z is a virtual one. Therefore, an outer circumference of the ocular guide 20 may be partially cut out. Note that the virtual region is not limited to the virtual circle Z, and may have any shape, provided that the any shape is one that is formed by projecting the ocular guide 20.

Moreover, the ocular guide 20 may be configured as a member separated from the assisting tool body 10, and may be directly connected to the eye drop expelling part 30a of the eye drop container 30 attached to the assisting tool body 10. In this case, how to connect the ocular guide 20 to the eye drop expelling part 30a is not limited in particular. For example, the cap put on the eye drop expelling part 30a of the eye drop container 30 is removed, and the ocular guide 20, instead of the cap, is fit on the eye drop expelling part 30a. This allows the ocular instillation assisting tool 1 to need merely the ocular guide 20 and not to need the assisting tool body 10, so that the ocular instillation assisting tool 1 has a simpler structure.

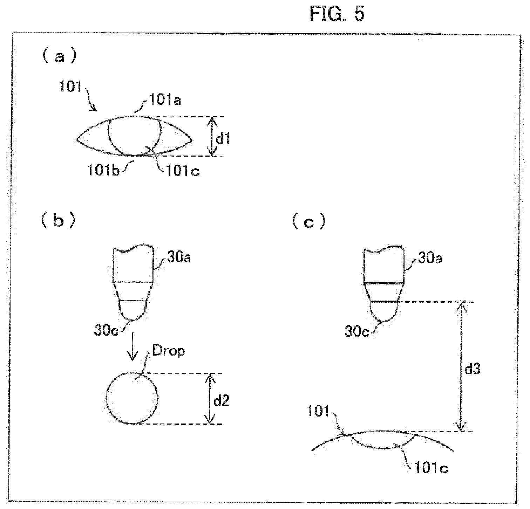

A success rate of the ocular instillation performed with use of the ocular instillation assisting tool 1 configured as described above will be described below with reference to Figs. 5 through 9.

<Ocular instillation experiment>

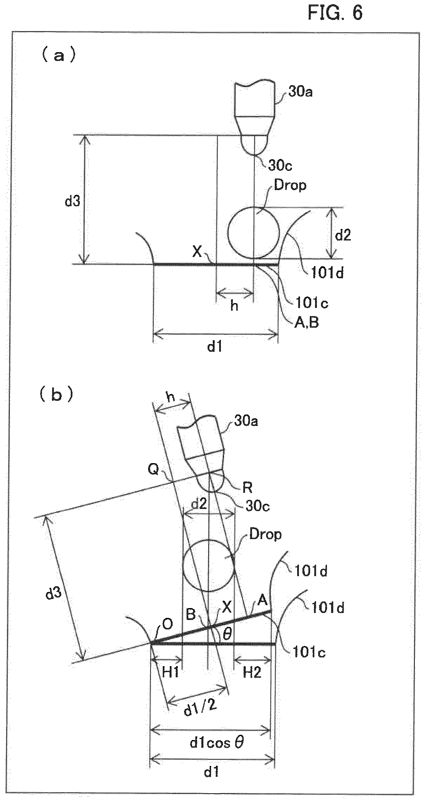

Fig. 5 is a drawing illustrating various conditions under which an ocular instillation experiment is carried out with use of the ocular instillation assisting tool 1. Fig. 6 is a drawing illustrating a success rate of ocular instillation which success rate depends on an ocular instillation distance and an ocular instillation angle that are employed in the ocular instillation experiment carried out with use of the ocular instillation assisting tool 1.

In a case where the ocular instillation experiment is carried out, a height d1 of an eye of a target of the ocular instillation (see (a) of Fig. 5), a drop diameter d2 of a drop of eye drops (see (b) of Fig. 5), and an ocular instillation distance d3 (see (c) of Fig. 5) are needed.

As illustrated in (a) of Fig. 5, the height d1 of the eye of the target is a width of a pupil (black eye) 101c between an upper eyelid 101a and a lower eyelid 101b of an eye 101. Here, the height d1 is assumed to be approximately 10 mm. A value of approximately 10 mm is derived from (i) the fact that a pupil 101c of an eye 101 of an ordinary Japanese has a diameter of approximately 12 mm and (ii) an assumption that parts of the pupil 101c which parts are respectively covered with the upper eyelid 101a and the lower eyelid 101b are each 1 mm.

As illustrated in (b) of Fig. 5, the drop diameter d2 is a diameter in a case where the drop which has dropped from the eye drop expelling part 30a has completely become a sphere. Here, the drop diameter d2 is assumed to be approximately 4 mm. A diameter of approximately 4 mm is a diameter in a case where the drop of 0.040 ml has completely become a sphere.

As illustrated in (c) of Fig. 5, the ocular instillation distance d3 is a distance from the expelling hole 30c of the eye drop expelling part 30a to a surface of the pupil 101c of the eye 101.

(a) of Fig. 6 illustrates a dropping state of the drop of the eye drops in a case where it is assumed that the target of the administration lies on his or her back (tilt angle $\theta$ = 0). (b) of Fig. 6 illustrates a dropping state of the drop of the eye drops in a case where it is assumed that the target of the administration looks up in the sitting or standing posture (tilt angle $\theta$ >0). Here, the pupil 101c illustrated in Fig. 6 is assumed to be the virtual circle Z illustrated in Fig. 1. The center of the pupil 101c is referred to as X, and a reached point which the expelling hole 30c of the eye drop expelling part 30a reaches if the expelling hole 30c is caused to linearly extend so as to reach the surface of the pupil 101c is referred to as A. Thus, as illustrated in (a) of Fig. 6, in a case where the tilt angle $\theta$ = 0, the reached point A on the pupil 101c is superposed on a dropped position B at which the drop drops. However, as illustrated in (a) of Fig. 6, in a case where the tilt angle $\theta$ > 0, the reached point A on the pupil 101c is shifted from the dropped position B at which the drop drops. That is, the dropped position B at which the drop drops is shifted toward the lower eyelid 101b on the pupil 101c. The tilt angle $\theta$ is an angle at which the surface of the pupil 101c tilts with respect to a plane perpendicular to a vertical direction.

Here, in (b) of Fig. 6, the following expressions are obtained with use of the ocular instillation distance d3, a shift amount h, and the tilt angle $\theta$.

$$\text{A vector } OX = d1/2(\cos\theta, \sin\theta)$$

$$\text{A vector } XQ = d3(\cos(\theta+\pi/2), \sin(\theta+\pi/2))$$

$$A \; vector \; QR \; = \; h(cos\theta, \; sin\theta)$$

Thus, a distance H1 from the lower eyelid to an edge of the drop on the pupil 101c can be expressed by the following expression (1).

$$H1 \; = \; ((d1/2)+h)cos\theta-d3sin\theta-d2/2 \; ...(1)$$

That is, H1 is a value obtained by subtracting a drop radius d2/2 of the drop from an X component of the vector OR.
Further, with use of the ocular instillation distance d3, the shift amount h, and the tilt angle θ, a distance H2 from the upper eyelid to an edge of the drop on the pupil 101c can be expressed by the following expression (2).

$$H2 \; = \; ((d1/2)-h)cos\theta+d3sin\theta-d2/2 \; ...(2)$$

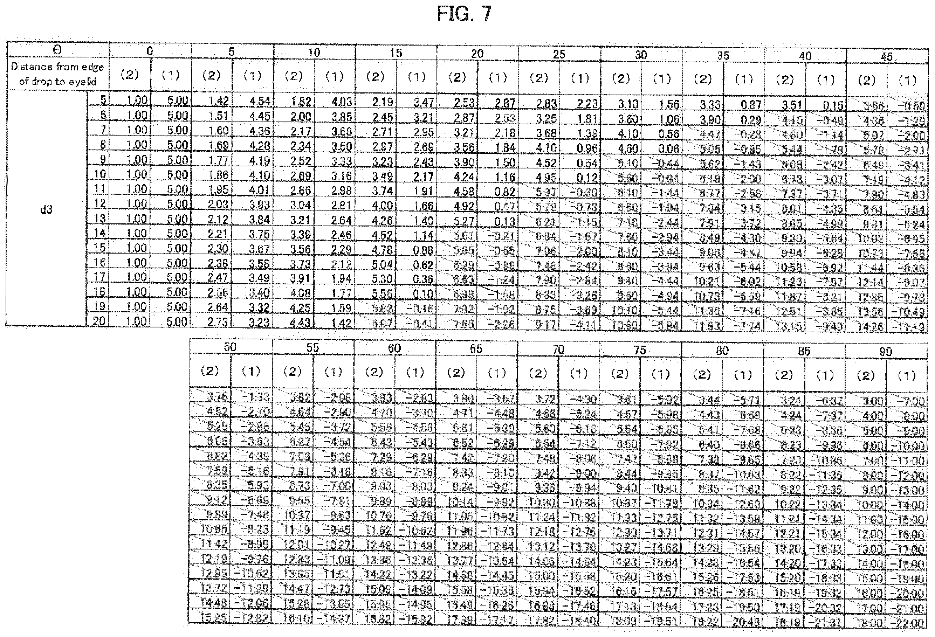

That is, H2 is a value obtained by subtracting the X component of the vector OR and the drop radius d2/2 of the drop from d1cosθ.
It is possible to know whether or not the ocular instillation has been successfully performed, from values obtained with use of the expressions (1) and (2).
Here, the experiment was carried out as follows. That is, the height d1 of the eye of the target was assumed to be approximately 10 mm. The drop diameter d2 was assumed to be approximately 4 mm. Then, values were calculated in accordance with of the expressions (1) and (2) while the ocular instillation distance d3 and the tilt angle θ were varied in value for each shift amount h between the reached point A and the center X. Figs. 7 through 9 each show experiment results.

<Experiment results>

Fig. 7 is a table showing values which were calculated in accordance with the expressions (1) and (2) while the tilt angle θ and the ocular instillation distance d3 were varied, in a case where the shift amount h was 2 mm. (1) and (2) in Fig. 7 correspond to the expressions (1) and (2), respectively.

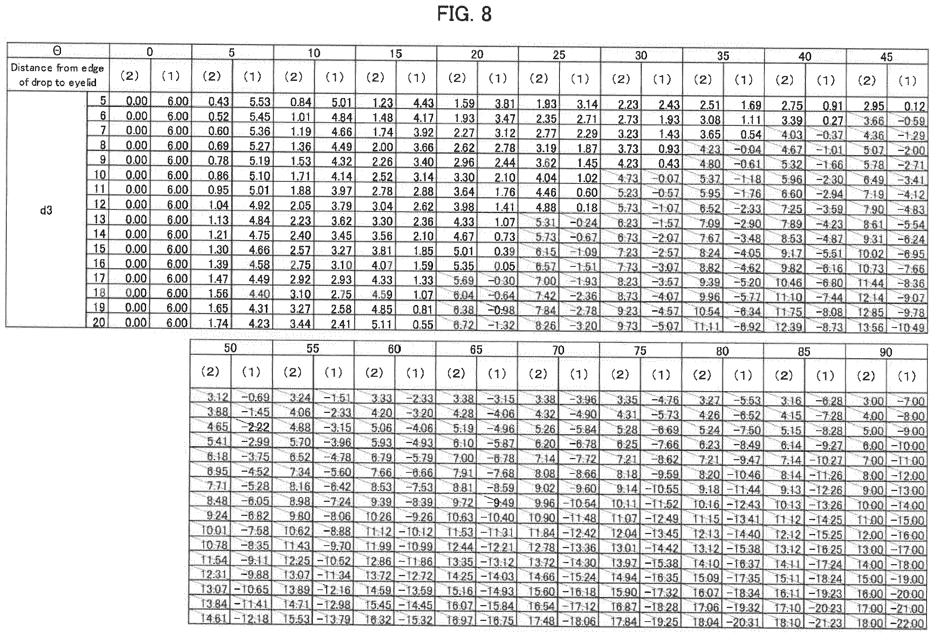

Fig. 8 is a table showing values which were calculated in accordance with the expressions (1) and (2) while the tilt angle θ and the ocular instillation distance d3 were varied, in a case where the shift amount h was 3 mm. (1) and (2) in Fig. 8 correspond to the expressions (1) and (2), respectively.

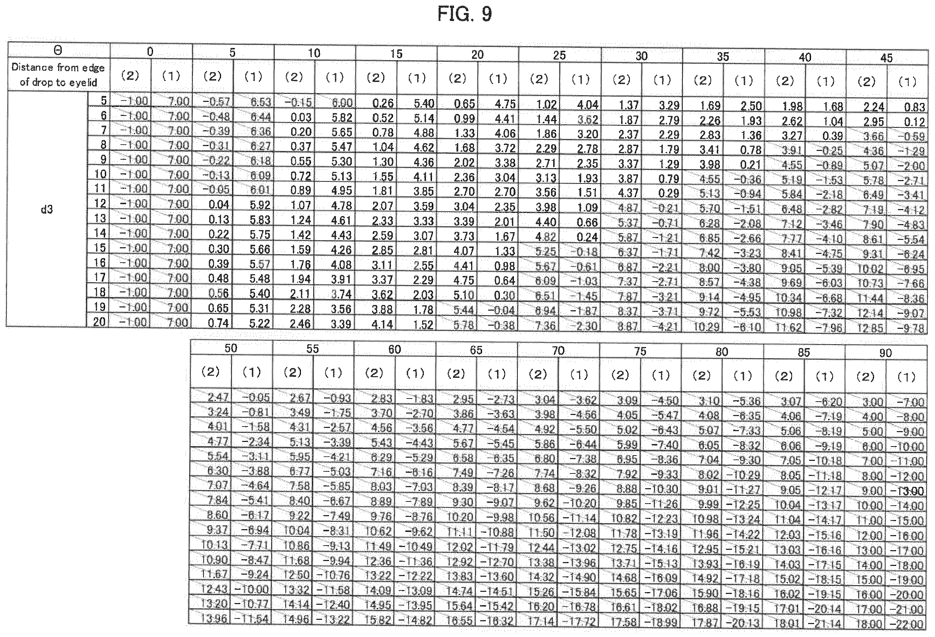

Fig. 9 is a table showing values which were calculated in accordance with the expressions (1) and (2) while the tilt angle θ and the ocular instillation distance d3 were varied, in a case where the shift amount h was 4 mm. (1) and (2) in Fig. 9 correspond to the expressions (1) and (2), respectively.
In a case where values which were calculated in accordance with the expressions (1) and (2) with use of a specific tilt angle θ shown in the table in Fig. 7 when the shift amount h was 2 mm (that is, the expelling hole of the eye drop expelling part 30a was shifted from the center of the pupil 101c toward the upper eyelid 101a by 2 mm) are both positive, this indicates that the ocular instillation was successfully performed. In the table, cells, other than cells through which diagonal lines are drawn, each indicate successful ocular instillation.
From the results, it is found that, even in a case where the ocular instillation is performed in a supine position (the tilt angle θ = 0°), the ocular instillation is successfully performed without the drop touching the upper eyelid 101a or eyelashes 101d. Moreover, in a case where the ocular instillation distance d3 is 5 mm, the ocular instillation is successfully performed at a tilt angle θ of 0° to 40°.
In a case where values which were calculated in accordance with the expressions (1) and (2) with use of a specific tilt angle θ shown in the table in Fig. 8 when the shift amount h was 3 mm (that is, the expelling hole of the eye drop expelling part 30a was shifted from the center of the pupil 101c toward the upper eyelid 101a by 3 mm) are both positive, this indicates that the ocular instillation was successfully carried out. In the table, cells, other than cells through which diagonal lines are drawn, each indicate successful ocular instillation.
From the results, it is found that, even in a case where the ocular instillation is performed in the supine position (the tilt angle θ = 0°), the ocular instillation is successfully performed without the drop touching the upper eyelid 101a or eyelashes 101d. Moreover, in a case where the ocular instillation distance d3 is 5 mm, the ocular instillation is successfully performed at a tilt angle θ of 0° to 45°.
In a case where values which were calculated in accordance with the expressions (1) and (2) with use of a specific tilt angle θ shown in the table in Fig. 9 when the shift amount h was 4 mm (that is, the expelling hole of the eye drop expelling part 30a was shifted from the center of the pupil 101c toward the upper eyelid 101a by 4 mm) are both positive, this indicates that the ocular instillation was successfully performed. In the table, cells, other than cells through which diagonal lines are drawn, each indicate successful ocular instillation.

From the results, it is found that, in a case where the ocular instillation is performed in the supine position (the tilt angle $\theta$ = 0°), the ocular instillation is not successfully performed because the drop touches the upper eyelid 101a or the eyelashes 101d. Therefore, it is found that, in a case where h = 4 mm, the ocular instillation is successfully performed by causing the ocular instillation distance d3 to be not less than 12 mm when the tilt angle $\theta$ is 5°.

As is clear from the above results, a preferable shift amount h is 2 mm and 3 mm in a case where the height d1 of the eye of the target is approximately 10 mm and the drop diameter d2 is approximately 4 mm. Note that, in a case where the shift amount h is 4 mm and the target of the administration lies on his or her back (tilt angle $\theta$ = 0), the eye drops touches the upper eyelid 101a or eyelash 101c. Therefore, the shift amount h of 4 mm is not preferable.

Note also that a particularly preferable shift amount h is 3 mm. This is because the shift amount h of 3 mm results in the successful ocular instillation at a wide tilt angle $\theta$, as compared with a case where the shift amount h is 2 mm.

Note that the preferable shift amount h is not limited to 3 mm. This is because the preferable shift amount h varies in a case where the height d1 of the eye of the target and/or the drop diameter d2 are/is varied.

<Effects>

As has been described, according to the ocular instillation assisting tool 1 having the above configuration, it is possible to appropriately perform ocular instillation, in either of a case where a target of the ocular instillation is in a sitting posture and a case where the target of the ocular instillation is in a standing posture. Since it is possible to accurately instill eye drops, an effect of an active ingredient of the eye drops is sufficiently exhibited. As a result, it is expected that (i) an eye disease is cured in a brief period of time, (ii) a mental burden on a patient is reduced, and (iii) a medical expense is significantly suppressed. Moreover, the ocular instillation assisting tool 1 has a simple structure. This allows production cost to be suppressed, and further allows the ocular instillation assisting tool 1 not to be easily break.

Medication management with use of the ocular instillation assisting tool 1 having the above configuration will be described below.

<Medication management>

An actual medication status of a patient can be grasped by incorporating a camera, a light emitting diode (LED) light, and/or a sensor into an ocular instillation device such as the ocular instillation assisting tool 1.

For example, in a case where an LED light and a camera are provided in the ocular guide 20 of the ocular instillation assisting tool 1, the LED light is turned on by a patient griping the ocular instillation assisting tool 1. This allows the patient not to close his or her eye, by causing the patient to perform ocular instillation while looking at the LED light. Further, when eye drops are expelled, an image of the eye is captured by the camera. Here, that the camera captures the image of the eye indicates that the camera captures an image of a region including the eye when the eye drops are expelled. For example, it is possible to capture a state immediately after the eye drops having been expelled are instilled into the eye, a state until the eye drops having been expelled are instilled into the eye, and the like. By accumulating images captured by the camera, it is also possible to manage an ocular instillation state of the patient.

Further, in a case where a sensor is provided to the ocular instillation assisting tool 1, it is possible to objectively detect whether or not the ocular instillation has been successfully performed, by employing an infrared temperature sensor. Specifically, an infrared sensor, such as one that is provided to Ocular Surface Thermographer (Tomey), is incorporated inside the ocular guide 20 of the ocular instillation assisting tool 1, and a change in temperature of a surface of the eye is measured for several seconds after the ocular instillation. Since a temperature of the eye drops is generally a room temperature whereas the temperature of the surface of the eye is approximately 37°C, a rapid fall of the temperature of the surface of the eye is detected at a moment of the ocular instillation. This phenomenon is utilized to make it possible to detect, with use of the infrared temperature sensor, whether or not the ocular instillation has been successfully performed.

Note that the temperature of the surface of the eye may be measured with use of a non-contact thermometer including a microsensor and an amplifier. In this case, since a response speed is as fast as 30 msec, it is possible to instantly detect the change in temperature of the surface of the eye when the drop enters the eye. This makes it possible to more quickly detect whether or not the ocular instillation has been successfully performed.

It is possible to grasp an actual medication status of the patient, by managing data obtained as a result of the ocular instillation with use of the ocular instillation assisting tool 1 (such as data indicative of an amount of the eye drops instilled into the eye, data on the image captured by the camera, and data indicative of whether or not the ocular instillation has been successfully performed) in association with a date of the ocular instillation.

Data as described above, which is obtained as a result of the ocular instillation, is, for example, stored in the ocular instillation assisting tool 200, which is an ocular instillation device, and is also synchronized with a smartphone and/or a personal computer. The data synchronized with the smartphone and/or the personal computer is automatically stored in a cloud server via a telephone line and/or the Internet. A specific medication management system will be described below.

<Medication management system>

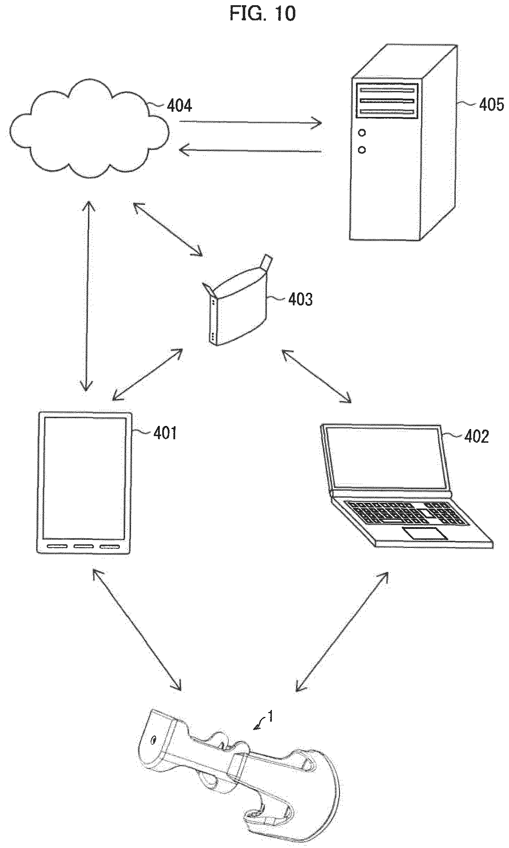

Fig. 10 is a schematic block diagram of a medication management system for management of ocular instillation performed with respect to a patient with use of the ocular instillation assisting tool 1 serving as an ocular instillation device.
As illustrated in Fig. 10, the medication management system, which employs the ocular instillation assisting tool 1, includes: a smartphone (mobile terminal) 401 which is operated by a manager (caretaker) who manages ocular instillation performed with respect to a user (patient) of the ocular instillation assisting tool 1; a personal computer 402 which is operated by another manager (doctor) who manages the ocular instillation performed with respect to the user (patient: target of administration); a router 403 connected to the Internet; a cloud computer 404; and a management server 405.

<Functions of ocular instillation assisting tool 1>

The ocular instillation assisting tool 1 is provided with a clock, a counter, a camera, a memory, an LED light, a battery, a battery indicator, a communication function (Bluetooth (registered trademark) 4.0, WiFi (registered trademark)), a bar code reading function, an IC tag reading function, and an alarm sounding function. These members are provided to the ocular instillation assisting tool 1 so that medication management is performed with use of the ocular instillation assisting tool 1 to which a drug product for the present invention is attached. This makes it possible to perform basic medication management as described below with use of the ocular instillation assisting tool 1.

(1) Determination of a kind of the drug product: A bar code attached to the eye drop container 30 containing the drug product for the present invention is read with use of the camera and the bar code reading function, and a kind of the drug product for the present invention is determined. The kind of the drug product for the present invention is automatically recorded when the eye drop container 30 is inserted into the ocular instillation assisting tool 1.
(2) Notification of a time for the ocular instillation (alarm): At a preset time for medication, an alarm is sounded with use of the alarm sounding function of the ocular instillation assisting tool 1. In this case, the alarm may be sounded by the smartphone 401 with use of which the ocular instillation assisting tool 1 is managed.
(3) Recording of a time of the ocular instillation: When the ocular instillation is performed with use of the ocular instillation assisting tool 1, (i) data on an image captured by the camera and (ii) data obtained from a sensor and indicative of whether or not the ocular instillation has been successfully performed are stored together with a date of the ocular instillation.
(4) An auxiliary light at a time of the ocular instillation: The LED light provided inside the ocular guide 20 is turned on with use of a switch provided to the ocular instillation assisting tool 1. This causes the patient to consciously open his or her eye, by causing the patient to perform the ocular instillation while looking at light from the LED light.
(5) Device ID management: Identification information is set for data management. The identification information is stored in the memory in association with obtained data.
(6) An ocular instillation calendar: Data (ocular instillation calendar) is created based on data having been stored. The data (ocular instillation calendar) shows records of the ocular instillation in a tabular form in chronological order. The records of the ocular instillation include the date of the ocular instillation, the kind of the drug product, the image data, and a note.
(7) Medication history data: A kind of a prescribed drug product and a history of medication (date of administration, dosage, and the like)
(8) Management of an amount of remaining eye drops: An amount of remaining eye drops in the container is managed by indication of (i) the amount of the remaining eye drops and (ii) a period of time for which the remaining eye drops can be used.
(9) Management of a remaining battery life of the ocular instillation assisting tool 1: A remaining battery life is indicated by a battery indicator. For example, in a case where a battery life is considered to be approximately one year, a warning is given one month before the end of the battery life.
(10) Data communication (Bluetooth (registered trademark) or WiFi (registered trademark)) with the smartphone 401 and/or the personal computer 402 is performed with use of the communication function.

<Functions that can be carried out by smartphone 401>

Use of functions of the smartphone 401 makes it possible to perform basic medication management as described below.

(1) Data communication (Bluetooth (registered trademark) or WiFi (registered trademark)) with the ocular instillation device is performed.
(2) Cloud data (medication management data) is downloaded from the cloud computer 404, and the medication management data thus downloaded is displayed.
(3) The medication management data is displayed on a calendar.
(4) The kind of the drug product in use, the amount of the drug product remaining, the number of remaining days for which the drug product can be used, and the like are displayed.
(5) The alarm is sounded at a time for administration of each of a plurality of kinds of drug products.
(6) The image data and the like which the ocular instillation assisting tool 200 has obtained is automatically uploaded to a cloud.

<Functions of personal computer 402>

Basically, the personal computer 402 can carry out the functions which the smartphone 401 can carry out. The personal computer 402 can carry out at least the following functions in addition to the functions which the smartphone 401 can carry out.

(1) Information on a plurality of patients, which information is stored in the cloud computer 404, is checked.
(2) Respective accumulated amounts of drug products prescribed for the patient who uses the ocular instillation assisting tool 1 are grasped.

<Actual example of medication management with use of medication management system>

(Operation of ocular instillation assisting tool 1)

A drug product recognizing method employed by the ocular instillation assisting tool 1 includes (i) a method of recognizing a drug product by software and (ii) a method of recognizing a drug product by hardware. Note that no electrical power switch is provided to the ocular instillation assisting tool 1 and a battery is mounted on the ocular instillation assisting tool 1, and that the ocular instillation assisting tool 1 is kept activated as long as electrical power is supplied.

(1) The method of recognizing a drug product by software

The camera mounted on the ocular instillation assisting tool 1 is directed to a bar code display section of a drug product. When reading of a bar code is completed, a beep is emitted. At this point of time, registration of a kind of the drug product is completed. The drug product whose kind has been registered is set in the ocular instillation assisting tool 1 such that a nozzle of a container of the drug product is appropriately positioned.

(2) The method of recognizing a drug product by hardware

The eye drop expelling part 30a of the eye drop container 30, which contains a drug product, has a raised portion. A position of the raised portion differs depending on a kind of the drug product. The eye drop container 30 containing the drug product is set in the ocular instillation assisting tool 1 such that a nozzle of the eye drop expelling part 30a is appropriately positioned. This allows recognition of the kind of the drug product contained in the eye drop container 30 thus set in the ocular instillation assisting tool 1, on the basis of the position of the raised portion provided to the eye drop expelling part 30a. Then, the kind of the drug product is registered in the ocular instillation assisting tool 1.
A user lightly grips the ocular instillation assisting tool 1 in which the eye drop container 30 containing the drug product, whose kind has been recognized by the above method (1) or (2), is set. The user then places the ocular guide 20 on a periphery of his or her eye. The ocular instillation assisting tool 1 is provided with the curved part 20f, in which a finger is put so that an eyelid is held down with the finger. The user puts his or her finger in the curved part 20f and then pulls his or her eyelid downward with the finger. In a case where the user holds the assisting tool body 10 of the ocular instillation assisting tool 1 in this state, the LED light is turned on. The patient pushes the eye drop container 30 attached to the assisting tool body 10 while looking at light from the LED light. This causes a drop of eye drops to be expelled from a leading end of the nozzle of the eye drop expelling part 30a.

Here, at the same time as the drop is expelled, a shutter of the camera provided in the ocular guide 20 is clicked and thereby a photograph is taken. In so doing, the shutter is instantaneously clicked a plurality of times, so that a plurality of photographs are taken. Alternatively, passage of the drop is detected by the sensor or a change in temperature of a surface of the eye at a time when the drop enters the eye is detected by the sensor. This makes it possible to monitor whether or not ocular instillation is successfully performed.

When the ocular instillation is finished, the eye drop container 30 containing the drug product is detached from the ocular instillation assisting tool 1. Then, a bar code of a next drug product is read so that the next drug product can be attached. Thereafter, ocular instillation is performed in the above-described manner. Subsequently, when ocular instillation of all drug products is finished, an ocular instillation operation is completed.

Subsequently, via Bluetooth (registered trademark) or WiFi (registered trademark), the ocular instillation assisting tool 1 synchronizes data with the smartphone 401 which a caretaker or the patient himself or herself operates.

(Operation of smartphone 401)

It is assumed that an application which is dedicated to the ocular instillation assisting tool 1 and which is used in the medication management system is installed in the smartphone 401.

Use of this application makes it possible for the smartphone 401 to communicate with the ocular instillation assisting tool 200. In a case where the smartphone 401 communicates with the ocular instillation assisting tool 1, the application in the smartphone obtains an ID number assigned to the ocular instillation assisting tool 1 so that the smartphone can recognize the ocular instillation assisting tool 1.

Further, use of the application makes it possible for the smartphone 401 to communicate with the management server 405 via the cloud computer 404, which management server 405 and cloud computer 404 are included in the medication management system, over telephone lines, by using the ID number assigned to the ocular instillation assisting tool 1 and a password.

In the application, a calendar function is set up. This makes it possible to register a prescribed drug product and to set a time for ocular instillation. In addition, the time for the ocular instillation can be set in the ocular instillation assisting tool 1 by synchronization with the ocular instillation assisting tool 1.

Use of the application makes it possible for the smartphone 401 to communicate with the ocular instillation assisting tool 1 and to take in data on a time of the ocular instillation, a kind of a drug product, an image(s) captured by the camera and/or the sensor, and the like. Not less than approximately 3 months' worth of data thus taken in can be stored in both of the ocular instillation assisting tool 1 and the smartphone 401. Even in a case where communication failure occurs, data is stored individually in each of the ocular instillation assisting tool 1 and the smartphone 401.

When the application is active, a calendar is displayed on a display screen of the smartphone 401. On the calendar, a mark is displayed which indicates whether or not ocular instillation of each drug product has been performed. Further, the image(s) and a record(s) which have been captured by the camera and/or the sensor can be checked as needed.

(Cloud server)

The cloud server is constructed with use of the cloud computer 404 and the management server 405 on the Internet, and has a database function and an analysis displaying function. Data for each day or data for a predetermined period is loaded into the cloud server via the smartphone 401. Such loading of the data accumulated in the smartphone 401 is carried out by clicking "Store" displayed on the display screen of the smartphone.

The cloud server can be accessed from the smartphone 401 or the personal computer 402 with use of the ID number of the ocular instillation assisting tool 200 and the password.

In a case where the cloud server is accessed from the smartphone 401, an access is allowed, based on a registered ID number, only to data on an individual associated with the ID number. In a case where a doctor desires to browse data on a plurality of patients, a right of access is separately set, and information on each of the plurality of patients is made only browsable. For example, on the personal computer 402, the doctor can check cloud data by causing the cloud data to be obtained and causing a calendar and image data, which show an ocular instillation profile of an individual, to be displayed based on the cloud data. Further, the doctor can check, on the personal computer 402, the number of times of ocular instillation of each drug product and an amount of the each drug product remaining, as additional information. When an amount of a remaining drug product becomes small, the doctor can find a display to move the doctor to prescribe.

The present invention is not limited to the embodiments, but can be altered by a skilled person in the art within the scope of the claims. The present invention also encompasses, in its technical scope, any embodiment derived by combining technical means disclosed in differing embodiments.

Reference Signs List

1 Ocular instillation assisting tool
10 Assisting tool body
10a First opening
10b Second opening
11 First rib
12 Second rib
13 Third rib
20 Ocular guide
20a First opening
20b Second opening
20c Third opening
20d Upper edge
20e Lower edge
20f Curved part
30 Eye drop container (container body)
30a Eye drop expelling part
30b Eye drop containing part
100 Patient (target of ocular instillation)
101 Eye
101a Upper eyelid
101b Lower eyelid
101c Pupil
101d Eyelash
102 Right hand
103 Left hand
200 Ocular instillation assisting tool
401 Smartphone
402 Personal computer
403 Router
404 Cloud computer
405 Management server

**Claims**

1. An ocular instillation assisting tool to which an eye drop container containing eye drops is detachably attached, said ocular instillation assisting tool comprising
a positioning member which is caused to abut on a periphery of an eye of a target of ocular instillation so that a position on the eye at which position a drop of the eye drops drops is determined,
the positioning member being formed so that, in a state where the target of the ocular instillation lies on his or her back and the positioning member abuts on the periphery of the eye, the drop of the eye drops contained in the eye drop container drops at a position which is shifted from a center of a surface of the eye toward an upper eyelid by a predetermined distance.

2. The ocular instillation assisting tool as set forth in claim 1, wherein a part of the positioning member which part faces a lower eyelid in a case where the positioning member is caused to abut on the periphery of the eye is cut out.

3. The ocular instillation assisting tool as set forth in claim 1 or 2, wherein parts of the positioning member which parts respectively abut on an outer corner and an inner corner of the eye in a case where the positioning member is caused to abut on the periphery of the eye are protruded toward the face of the target of the ocular instillation, as compared with the other parts.

4. The ocular instillation assisting tool as set forth in any one of claims 1 through 3, further comprising
an attachment member which is connected to the positioning member and to which the eye drop container is detachably attached,
the attachment member having (i) a first opening through which a container body of the eye drop container is attached to and detached from the attachment member and (ii) at a position which faces the first opening, a second opening through which the container body of the eye drop container that is attached to the attachment member is partially exposed.

5. An ocular instillation assisting tool to which an eye drop container containing eye drops is detachably attached, said ocular instillation assisting tool comprising:

a positioning member which is caused to abut on a periphery of an eye of a target of ocular instillation so that a position on the eye at which position a drop of the eye drops drops is determined; and
an attachment member which is connected to the positioning member and to which the eye drop container is detachably attached,
assuming that, in a state where the target of the ocular instillation lies on his or her back and the positioning member abuts on the periphery of the eye, a part of the positioning member which part abuts on an upper eyelid is located on an upper side, a first point being shifted from a second point toward the upper side by a predetermined distance, the first point being obtained by projecting, on a bottom surface of the attachment member, a point which is obtained by projecting, on a first region obtained by projecting the positioning member, an expelling hole of the eye drop container which is attached to the attachment member, the second point being obtained by projecting a center of the first region on the bottom surface, the expelling hole being a hole through which the drop of the eye drops is expelled.

6. The ocular instillation assisting tool as set forth in claim 5, wherein the predetermined distance is 2 mm to 4 mm.

FIG. 1

FIG. 2

FIG. 3

FIG. 4

## FIG. 5

(a)

101
101a
101b
101c
d1

(b)

30a
30c
Drop
d2

(c)

30a
30c
d3
101
101c

FIG. 6

(a)

(b)

## FIG. 7

EP 3 766 472 A1

| Θ / Distance from edge of drop to eyelid | 0 (2) | 0 (1) | 5 (2) | 5 (1) | 10 (2) | 10 (1) | 15 (2) | 15 (1) | 20 (2) | 20 (1) | 25 (2) | 25 (1) | 30 (2) | 30 (1) | 35 (2) | 35 (1) | 40 (2) | 40 (1) | 45 (2) | 45 (1) |
|---|---|---|---|---|---|---|---|---|---|---|---|---|---|---|---|---|---|---|---|---|
| 5 (d3) | 1.00 | 5.00 | 1.42 | 4.54 | 1.82 | 4.03 | 2.19 | 3.47 | 2.53 | 2.87 | 2.83 | 2.23 | 3.10 | 1.56 | 3.33 | 0.87 | 3.51 | 0.15 | 3.66 | -0.59 |
| 6 | 1.00 | 5.00 | 1.51 | 4.45 | 2.00 | 3.85 | 2.45 | 3.21 | 2.87 | 2.53 | 3.25 | 1.81 | 3.60 | 1.06 | 3.90 | 0.29 | 4.15 | -0.49 | 4.36 | -1.29 |
| 7 | 1.00 | 5.00 | 1.60 | 4.36 | 2.17 | 3.68 | 2.71 | 2.95 | 3.21 | 2.18 | 3.68 | 1.39 | 4.10 | 0.56 | 4.47 | -0.28 | 4.80 | -1.14 | 5.07 | -2.00 |
| 8 | 1.00 | 5.00 | 1.69 | 4.28 | 2.34 | 3.50 | 2.97 | 2.69 | 3.56 | 1.84 | 4.10 | 0.96 | 4.60 | 0.06 | 5.05 | -0.85 | 5.44 | -1.78 | 5.78 | -2.71 |
| 9 | 1.00 | 5.00 | 1.77 | 4.19 | 2.52 | 3.33 | 3.23 | 2.43 | 3.90 | 1.50 | 4.52 | 0.54 | 5.10 | -0.44 | 5.62 | -1.43 | 6.08 | -2.42 | 6.49 | -3.41 |
| 10 | 1.00 | 5.00 | 1.86 | 4.10 | 2.69 | 3.16 | 3.49 | 2.17 | 4.24 | 1.16 | 4.95 | 0.12 | 5.60 | -0.94 | 6.19 | -2.00 | 6.73 | -3.07 | 7.19 | -4.12 |
| 11 | 1.00 | 5.00 | 1.95 | 4.01 | 2.86 | 2.98 | 3.74 | 1.91 | 4.58 | 0.82 | 5.37 | -0.30 | 6.10 | -1.44 | 6.77 | -2.58 | 7.37 | -3.71 | 7.90 | -4.83 |
| 12 | 1.00 | 5.00 | 2.03 | 3.93 | 3.04 | 2.81 | 4.00 | 1.66 | 4.92 | 0.47 | 5.79 | -0.73 | 6.60 | -1.94 | 7.34 | -3.15 | 8.01 | -4.35 | 8.61 | -5.54 |
| 13 | 1.00 | 5.00 | 2.12 | 3.84 | 3.21 | 2.64 | 4.26 | 1.40 | 5.27 | 0.13 | 6.21 | -1.15 | 7.10 | -2.44 | 7.91 | -3.72 | 8.65 | -4.99 | 9.31 | -6.24 |
| 14 | 1.00 | 5.00 | 2.21 | 3.75 | 3.39 | 2.46 | 4.52 | 1.14 | 5.61 | -0.21 | 6.64 | -1.57 | 7.60 | -2.94 | 8.49 | -4.30 | 9.30 | -5.64 | 10.02 | -6.95 |
| 15 | 1.00 | 5.00 | 2.30 | 3.67 | 3.56 | 2.29 | 4.78 | 0.88 | 5.95 | -0.55 | 7.06 | -2.00 | 8.10 | -3.44 | 9.06 | -4.87 | 9.94 | -6.28 | 10.73 | -7.66 |
| 16 | 1.00 | 5.00 | 2.38 | 3.58 | 3.73 | 2.12 | 5.04 | 0.62 | 6.29 | -0.89 | 7.48 | -2.42 | 8.60 | -3.94 | 9.63 | -5.44 | 10.58 | -6.92 | 11.44 | -8.36 |
| 17 | 1.00 | 5.00 | 2.47 | 3.49 | 3.91 | 1.94 | 5.30 | 0.36 | 6.63 | -1.24 | 7.90 | -2.84 | 9.10 | -4.44 | 10.21 | -6.02 | 11.23 | -7.57 | 12.14 | -9.07 |
| 18 | 1.00 | 5.00 | 2.56 | 3.40 | 4.08 | 1.77 | 5.56 | 0.10 | 6.98 | -1.58 | 8.33 | -3.26 | 9.60 | -4.94 | 10.78 | -6.59 | 11.87 | -8.21 | 12.85 | -9.78 |
| 19 | 1.00 | 5.00 | 2.64 | 3.32 | 4.25 | 1.59 | 5.82 | -0.16 | 7.32 | -1.92 | 8.75 | -3.69 | 10.10 | -5.44 | 11.36 | -7.16 | 12.51 | -8.85 | 13.56 | -10.49 |
| 20 | 1.00 | 5.00 | 2.73 | 3.23 | 4.43 | 1.42 | 6.07 | -0.41 | 7.66 | -2.26 | 9.17 | -4.11 | 10.60 | -5.94 | 11.93 | -7.74 | 13.15 | -9.49 | 14.26 | -11.19 |

| 50 (2) | 50 (1) | 55 (2) | 55 (1) | 60 (2) | 60 (1) | 65 (2) | 65 (1) | 70 (2) | 70 (1) | 75 (2) | 75 (1) | 80 (2) | 80 (1) | 85 (2) | 85 (1) | 90 (2) | 90 (1) |
|---|---|---|---|---|---|---|---|---|---|---|---|---|---|---|---|---|---|
| 3.76 | -1.33 | 3.82 | -2.08 | 3.83 | -2.83 | 3.80 | -3.57 | 3.72 | -4.30 | 3.61 | -5.02 | 3.44 | -5.71 | 3.24 | -6.37 | 3.00 | -7.00 |
| 4.52 | -2.10 | 4.64 | -2.90 | 4.70 | -3.70 | 4.71 | -4.48 | 4.66 | -5.24 | 4.57 | -5.98 | 4.43 | -6.69 | 4.24 | -7.37 | 4.00 | -8.00 |
| 5.29 | -2.86 | 5.45 | -3.72 | 5.56 | -4.56 | 5.61 | -5.39 | 5.60 | -6.18 | 5.54 | -6.95 | 5.41 | -7.68 | 5.23 | -8.36 | 5.00 | -9.00 |
| 6.06 | -3.63 | 6.27 | -4.54 | 6.43 | -5.43 | 6.52 | -6.29 | 6.54 | -7.12 | 6.50 | -7.92 | 6.40 | -8.66 | 6.23 | -9.36 | 6.00 | -10.00 |
| 6.82 | -4.39 | 7.09 | -5.36 | 7.29 | -6.29 | 7.42 | -7.20 | 7.48 | -8.06 | 7.47 | -8.88 | 7.38 | -9.65 | 7.23 | -10.36 | 7.00 | -11.00 |
| 7.59 | -5.16 | 7.91 | -6.18 | 8.16 | -7.16 | 8.33 | -8.10 | 8.42 | -9.00 | 8.44 | -9.85 | 8.37 | -10.63 | 8.22 | -11.35 | 8.00 | -12.00 |
| 8.35 | -5.93 | 8.73 | -7.00 | 9.03 | -8.03 | 9.24 | -9.01 | 9.36 | -9.94 | 9.40 | -10.81 | 9.35 | -11.62 | 9.22 | -12.35 | 9.00 | -13.00 |
| 9.12 | -6.69 | 9.55 | -7.81 | 9.89 | -8.89 | 10.14 | -9.92 | 10.30 | -10.88 | 10.37 | -11.78 | 10.34 | -12.60 | 10.22 | -13.34 | 10.00 | -14.00 |
| 9.89 | -7.46 | 10.37 | -8.63 | 10.76 | -9.76 | 11.05 | -10.82 | 11.24 | -11.82 | 11.33 | -12.75 | 11.32 | -13.59 | 11.21 | -14.34 | 11.00 | -15.00 |
| 10.65 | -8.23 | 11.19 | -9.45 | 11.62 | -10.62 | 11.96 | -11.73 | 12.18 | -12.76 | 12.30 | -13.71 | 12.31 | -14.57 | 12.21 | -15.34 | 12.00 | -16.00 |
| 11.42 | -8.99 | 12.01 | -10.27 | 12.49 | -11.49 | 12.86 | -12.64 | 13.12 | -13.70 | 13.27 | -14.68 | 13.29 | -15.56 | 13.20 | -16.33 | 13.00 | -17.00 |
| 12.19 | -9.76 | 12.83 | -11.09 | 13.36 | -12.36 | 13.77 | -13.54 | 14.06 | -14.64 | 14.23 | -15.64 | 14.28 | -16.54 | 14.20 | -17.33 | 14.00 | -18.00 |
| 12.95 | -10.52 | 13.65 | -11.91 | 14.22 | -13.22 | 14.68 | -14.45 | 15.00 | -15.58 | 15.20 | -16.61 | 15.26 | -17.53 | 15.20 | -18.33 | 15.00 | -19.00 |
| 13.72 | -11.29 | 14.47 | -12.73 | 15.09 | -14.09 | 15.58 | -15.36 | 15.94 | -16.52 | 16.16 | -17.57 | 16.25 | -18.51 | 16.19 | -19.32 | 16.00 | -20.00 |
| 14.48 | -12.06 | 15.28 | -13.55 | 15.95 | -14.95 | 16.49 | -16.26 | 16.88 | -17.46 | 17.13 | -18.54 | 17.23 | -19.50 | 17.19 | -20.32 | 17.00 | -21.00 |
| 15.25 | -12.82 | 16.10 | -14.37 | 16.82 | -15.82 | 17.39 | -17.17 | 17.82 | -18.40 | 18.09 | -19.51 | 18.22 | -20.48 | 18.19 | -21.31 | 18.00 | -22.00 |

# FIG. 8

| Θ | | 0 | | 5 | | 10 | | 15 | | 20 | | 25 | | 30 | | 35 | | 40 | | 45 | |
|---|---|---|---|---|---|---|---|---|---|---|---|---|---|---|---|---|---|---|---|---|
| Distance from edge of drop to eyelid | | (2) | (1) | (2) | (1) | (2) | (1) | (2) | (1) | (2) | (1) | (2) | (1) | (2) | (1) | (2) | (1) | (2) | (1) | (2) | (1) |
| d3 | 5 | 0.00 | 6.00 | 0.43 | 5.53 | 0.84 | 5.01 | 1.23 | 4.43 | 1.59 | 3.81 | 1.93 | 3.14 | 2.23 | 2.43 | 2.51 | 1.69 | 2.75 | 0.91 | 2.95 | 0.12 |
| | 6 | 0.00 | 6.00 | 0.52 | 5.45 | 1.01 | 4.84 | 1.48 | 4.17 | 1.93 | 3.47 | 2.35 | 2.71 | 2.73 | 1.93 | 3.08 | 1.11 | 3.39 | 0.27 | 3.66 | -0.59 |
| | 7 | 0.00 | 6.00 | 0.60 | 5.36 | 1.19 | 4.66 | 1.74 | 3.92 | 2.27 | 3.12 | 2.77 | 2.29 | 3.23 | 1.43 | 3.65 | 0.54 | 4.03 | -0.37 | 4.36 | -1.29 |
| | 8 | 0.00 | 6.00 | 0.69 | 5.27 | 1.36 | 4.49 | 2.00 | 3.66 | 2.62 | 2.78 | 3.19 | 1.87 | 3.73 | 0.93 | 4.23 | -0.04 | 4.67 | -1.01 | 5.07 | -2.00 |
| | 9 | 0.00 | 6.00 | 0.78 | 5.19 | 1.53 | 4.32 | 2.26 | 3.40 | 2.96 | 2.44 | 3.62 | 1.45 | 4.23 | 0.43 | 4.80 | -0.61 | 5.32 | -1.66 | 5.78 | -2.71 |
| | 10 | 0.00 | 6.00 | 0.86 | 5.10 | 1.71 | 4.14 | 2.52 | 3.14 | 3.30 | 2.10 | 4.04 | 1.02 | 4.73 | -0.07 | 5.37 | -1.18 | 5.96 | -2.30 | 6.49 | -3.41 |
| | 11 | 0.00 | 6.00 | 0.95 | 5.01 | 1.88 | 3.97 | 2.78 | 2.88 | 3.64 | 1.76 | 4.46 | 0.60 | 5.23 | -0.57 | 5.95 | -1.76 | 6.60 | -2.94 | 7.19 | -4.12 |
| | 12 | 0.00 | 6.00 | 1.04 | 4.92 | 2.05 | 3.79 | 3.04 | 2.62 | 3.98 | 1.41 | 4.88 | 0.18 | 5.73 | -1.07 | 6.52 | -2.33 | 7.25 | -3.59 | 7.90 | -4.83 |
| | 13 | 0.00 | 6.00 | 1.13 | 4.84 | 2.23 | 3.62 | 3.30 | 2.36 | 4.33 | 1.07 | 5.31 | -0.24 | 6.23 | -1.57 | 7.09 | -2.90 | 7.89 | -4.23 | 8.61 | -5.54 |
| | 14 | 0.00 | 6.00 | 1.21 | 4.75 | 2.40 | 3.45 | 3.56 | 2.10 | 4.67 | 0.73 | 5.73 | -0.67 | 6.73 | -2.07 | 7.67 | -3.48 | 8.53 | -4.87 | 9.31 | -6.24 |
| | 15 | 0.00 | 6.00 | 1.30 | 4.66 | 2.57 | 3.27 | 3.81 | 1.85 | 5.01 | 0.39 | 6.15 | -1.09 | 7.23 | -2.57 | 8.24 | -4.05 | 9.17 | -5.51 | 10.02 | -6.95 |
| | 16 | 0.00 | 6.00 | 1.39 | 4.58 | 2.75 | 3.10 | 4.07 | 1.59 | 5.35 | 0.05 | 6.57 | -1.51 | 7.73 | -3.07 | 8.82 | -4.62 | 9.82 | -6.16 | 10.73 | -7.66 |
| | 17 | 0.00 | 6.00 | 1.47 | 4.49 | 2.92 | 2.93 | 4.33 | 1.33 | 5.69 | -0.30 | 7.00 | -1.93 | 8.23 | -3.57 | 9.39 | -5.20 | 10.46 | -6.80 | 11.44 | -8.36 |
| | 18 | 0.00 | 6.00 | 1.56 | 4.40 | 3.10 | 2.75 | 4.59 | 1.07 | 6.04 | -0.64 | 7.42 | -2.36 | 8.73 | -4.07 | 9.96 | -5.77 | 11.10 | -7.44 | 12.14 | -9.07 |
| | 19 | 0.00 | 6.00 | 1.65 | 4.31 | 3.27 | 2.58 | 4.85 | 0.81 | 6.38 | -0.98 | 7.84 | -2.78 | 9.23 | -4.57 | 10.54 | -6.34 | 11.75 | -8.08 | 12.85 | -9.78 |
| | 20 | 0.00 | 6.00 | 1.74 | 4.23 | 3.44 | 2.41 | 5.11 | 0.55 | 6.72 | -1.32 | 8.26 | -3.20 | 9.73 | -5.07 | 11.11 | -6.92 | 12.39 | -8.73 | 13.56 | -10.49 |

| 50 | | 55 | | 60 | | 65 | | 70 | | 75 | | 80 | | 85 | | 90 | |
|---|---|---|---|---|---|---|---|---|---|---|---|---|---|---|---|---|---|
| (2) | (1) | (2) | (1) | (2) | (1) | (2) | (1) | (2) | (1) | (2) | (1) | (2) | (1) | (2) | (1) | (2) | (1) |
| 3.12 | -0.69 | 3.24 | -1.51 | 3.33 | -2.33 | 3.38 | -3.15 | 3.38 | -3.96 | 3.35 | -4.76 | 3.27 | -5.53 | 3.16 | -6.28 | 3.00 | -7.00 |
| 3.88 | -1.45 | 4.06 | -2.33 | 4.20 | -3.20 | 4.28 | -4.06 | 4.32 | -4.90 | 4.31 | -5.73 | 4.26 | -6.52 | 4.15 | -7.28 | 4.00 | -8.00 |
| 4.65 | -2.22 | 4.88 | -3.15 | 5.06 | -4.06 | 5.19 | -4.96 | 5.26 | -5.84 | 5.28 | -6.69 | 5.24 | -7.50 | 5.15 | -8.28 | 5.00 | -9.00 |
| 5.41 | -2.99 | 5.70 | -3.96 | 5.93 | -4.93 | 6.10 | -5.87 | 6.20 | -6.78 | 6.25 | -7.66 | 6.23 | -8.49 | 6.14 | -9.27 | 6.00 | -10.00 |
| 6.18 | -3.75 | 6.52 | -4.78 | 6.79 | -5.79 | 7.00 | -6.78 | 7.14 | -7.72 | 7.21 | -8.62 | 7.21 | -9.47 | 7.14 | -10.27 | 7.00 | -11.00 |
| 6.95 | -4.52 | 7.34 | -5.60 | 7.66 | -6.66 | 7.91 | -7.68 | 8.08 | -8.66 | 8.18 | -9.59 | 8.20 | -10.46 | 8.14 | -11.26 | 8.00 | -12.00 |
| 7.71 | -5.28 | 8.16 | -6.42 | 8.53 | -7.53 | 8.81 | -8.59 | 9.02 | -9.60 | 9.14 | -10.55 | 9.18 | -11.44 | 9.13 | -12.26 | 9.00 | -13.00 |
| 8.48 | -6.05 | 8.98 | -7.24 | 9.39 | -8.39 | 9.72 | -9.49 | 9.96 | -10.54 | 10.11 | -11.52 | 10.16 | -12.43 | 10.13 | -13.26 | 10.00 | -14.00 |
| 9.24 | -6.82 | 9.80 | -8.06 | 10.26 | -9.26 | 10.63 | -10.40 | 10.90 | -11.48 | 11.07 | -12.49 | 11.15 | -13.41 | 11.12 | -14.25 | 11.00 | -15.00 |
| 10.01 | -7.58 | 10.62 | -8.88 | 11.12 | -10.12 | 11.53 | -11.31 | 11.84 | -12.42 | 12.04 | -13.45 | 12.13 | -14.40 | 12.12 | -15.25 | 12.00 | -16.00 |
| 10.78 | -8.35 | 11.43 | -9.70 | 11.99 | -10.99 | 12.44 | -12.21 | 12.78 | -13.36 | 13.01 | -14.42 | 13.12 | -15.38 | 13.12 | -16.25 | 13.00 | -17.00 |
| 11.54 | -9.11 | 12.25 | -10.52 | 12.86 | -11.86 | 13.35 | -13.12 | 13.72 | -14.30 | 13.97 | -15.38 | 14.10 | -16.37 | 14.11 | -17.24 | 14.00 | -18.00 |
| 12.31 | -9.88 | 13.07 | -11.34 | 13.72 | -12.72 | 14.25 | -14.03 | 14.66 | -15.24 | 14.94 | -16.35 | 15.09 | -17.35 | 15.11 | -18.24 | 15.00 | -19.00 |
| 13.07 | -10.65 | 13.89 | -12.16 | 14.59 | -13.59 | 15.16 | -14.93 | 15.60 | -16.18 | 15.90 | -17.32 | 16.07 | -18.34 | 16.11 | -19.23 | 16.00 | -20.00 |
| 13.84 | -11.41 | 14.71 | -12.98 | 15.45 | -14.45 | 16.07 | -15.84 | 16.54 | -17.12 | 16.87 | -18.28 | 17.06 | -19.32 | 17.10 | -20.23 | 17.00 | -21.00 |
| 14.61 | -12.18 | 15.53 | -13.79 | 16.32 | -15.32 | 16.97 | -16.75 | 17.48 | -18.06 | 17.84 | -19.25 | 18.04 | -20.31 | 18.10 | -21.23 | 18.00 | -22.00 |

EP 3 766 472 A1

# FIG. 9

| Θ | | 0 | | 5 | | 10 | | 15 | | 20 | | 25 | | 30 | | 35 | | 40 | | 45 | |
|---|---|---|---|---|---|---|---|---|---|---|---|---|---|---|---|---|---|---|---|---|
| Distance from edge of drop to eyelid | | (2) | (1) | (2) | (1) | (2) | (1) | (2) | (1) | (2) | (1) | (2) | (1) | (2) | (1) | (2) | (1) | (2) | (1) | (2) | (1) |
| d3 | 5 | -1.00 | 7.00 | -0.57 | 6.53 | -0.15 | 6.00 | 0.26 | 5.40 | 0.65 | 4.75 | 1.02 | 4.04 | 1.37 | 3.29 | 1.69 | 2.50 | 1.98 | 1.68 | 2.24 | 0.83 |
| | 6 | -1.00 | 7.00 | -0.48 | 6.44 | 0.03 | 5.82 | 0.52 | 5.14 | 0.99 | 4.41 | 1.44 | 3.62 | 1.87 | 2.79 | 2.26 | 1.93 | 2.62 | 1.04 | 2.95 | 0.12 |
| | 7 | -1.00 | 7.00 | -0.39 | 6.36 | 0.20 | 5.65 | 0.78 | 4.88 | 1.33 | 4.06 | 1.86 | 3.20 | 2.37 | 2.29 | 2.83 | 1.36 | 3.27 | 0.39 | 3.66 | -0.59 |
| | 8 | -1.00 | 7.00 | -0.31 | 6.27 | 0.37 | 5.47 | 1.04 | 4.62 | 1.68 | 3.72 | 2.29 | 2.78 | 2.87 | 1.79 | 3.41 | 0.78 | 3.91 | -0.25 | 4.36 | -1.29 |
| | 9 | -1.00 | 7.00 | -0.22 | 6.18 | 0.55 | 5.30 | 1.30 | 4.36 | 2.02 | 3.38 | 2.71 | 2.35 | 3.37 | 1.29 | 3.98 | 0.21 | 4.55 | -0.89 | 5.07 | -2.00 |
| | 10 | -1.00 | 7.00 | -0.13 | 6.09 | 0.72 | 5.13 | 1.55 | 4.11 | 2.36 | 3.04 | 3.13 | 1.93 | 3.87 | 0.79 | 4.55 | -0.36 | 5.19 | -1.53 | 5.78 | -2.71 |
| | 11 | -1.00 | 7.00 | -0.05 | 6.01 | 0.89 | 4.95 | 1.81 | 3.85 | 2.70 | 2.70 | 3.56 | 1.51 | 4.37 | 0.29 | 5.13 | -0.94 | 5.84 | -2.18 | 6.49 | -3.41 |
| | 12 | -1.00 | 7.00 | 0.04 | 5.92 | 1.07 | 4.78 | 2.07 | 3.59 | 3.04 | 2.35 | 3.98 | 1.09 | 4.87 | -0.21 | 5.70 | -1.51 | 6.48 | -2.82 | 7.19 | -4.12 |
| | 13 | -1.00 | 7.00 | 0.13 | 5.83 | 1.24 | 4.61 | 2.33 | 3.33 | 3.39 | 2.01 | 4.40 | 0.66 | 5.37 | -0.71 | 6.28 | -2.08 | 7.12 | -3.46 | 7.90 | -4.83 |
| | 14 | -1.00 | 7.00 | 0.22 | 5.75 | 1.42 | 4.43 | 2.59 | 3.07 | 3.73 | 1.67 | 4.82 | 0.24 | 5.87 | -1.21 | 6.85 | -2.66 | 7.77 | -4.10 | 8.61 | -5.54 |
| | 15 | -1.00 | 7.00 | 0.30 | 5.66 | 1.59 | 4.26 | 2.85 | 2.81 | 4.07 | 1.33 | 5.25 | -0.18 | 6.37 | -1.71 | 7.42 | -3.23 | 8.41 | -4.75 | 9.31 | -6.24 |
| | 16 | -1.00 | 7.00 | 0.39 | 5.57 | 1.76 | 4.08 | 3.11 | 2.55 | 4.41 | 0.98 | 5.67 | -0.61 | 6.87 | -2.21 | 8.00 | -3.80 | 9.05 | -5.39 | 10.02 | -6.95 |
| | 17 | -1.00 | 7.00 | 0.48 | 5.48 | 1.94 | 3.91 | 3.37 | 2.29 | 4.75 | 0.64 | 6.09 | -1.03 | 7.37 | -2.71 | 8.57 | -4.38 | 9.69 | -6.03 | 10.73 | -7.66 |
| | 18 | -1.00 | 7.00 | 0.56 | 5.40 | 2.11 | 3.74 | 3.62 | 2.03 | 5.10 | 0.30 | 6.51 | -1.45 | 7.87 | -3.21 | 9.14 | -4.95 | 10.34 | -6.68 | 11.44 | -8.36 |
| | 19 | -1.00 | 7.00 | 0.65 | 5.31 | 2.28 | 3.56 | 3.88 | 1.78 | 5.44 | -0.04 | 6.94 | -1.87 | 8.37 | -3.71 | 9.72 | -5.53 | 10.98 | -7.32 | 12.14 | -9.07 |
| | 20 | -1.00 | 7.00 | 0.74 | 5.22 | 2.46 | 3.39 | 4.14 | 1.52 | 5.78 | -0.38 | 7.36 | -2.30 | 8.87 | -4.21 | 10.29 | -6.10 | 11.62 | -7.96 | 12.85 | -9.78 |

| 50 | | 55 | | 60 | | 65 | | 70 | | 75 | | 80 | | 85 | | 90 | |
|---|---|---|---|---|---|---|---|---|---|---|---|---|---|---|---|---|---|
| (2) | (1) | (2) | (1) | (2) | (1) | (2) | (1) | (2) | (1) | (2) | (1) | (2) | (1) | (2) | (1) | (2) | (1) |
| 2.47 | -0.05 | 2.67 | -0.93 | 2.83 | -1.83 | 2.95 | -2.73 | 3.04 | -3.62 | 3.09 | -4.50 | 3.10 | -5.36 | 3.07 | -6.20 | 3.00 | -7.00 |
| 3.24 | -0.81 | 3.49 | -1.75 | 3.70 | -2.70 | 3.86 | -3.63 | 3.98 | -4.56 | 4.05 | -5.47 | 4.08 | -6.35 | 4.06 | -7.19 | 4.00 | -8.00 |
| 4.01 | -1.58 | 4.31 | -2.57 | 4.56 | -3.56 | 4.77 | -4.54 | 4.92 | -5.50 | 5.02 | -6.43 | 5.07 | -7.33 | 5.06 | -8.19 | 5.00 | -9.00 |
| 4.77 | -2.34 | 5.13 | -3.39 | 5.43 | -4.43 | 5.67 | -5.45 | 5.86 | -6.44 | 5.99 | -7.40 | 6.05 | -8.32 | 6.06 | -9.19 | 6.00 | -10.00 |
| 5.54 | -3.11 | 5.95 | -4.21 | 6.29 | -5.29 | 6.58 | -6.35 | 6.80 | -7.38 | 6.95 | -8.36 | 7.04 | -9.30 | 7.05 | -10.18 | 7.00 | -11.00 |
| 6.30 | -3.88 | 6.77 | -5.03 | 7.16 | -6.16 | 7.49 | -7.26 | 7.74 | -8.32 | 7.92 | -9.33 | 8.02 | -10.29 | 8.05 | -11.18 | 8.00 | -12.00 |
| 7.07 | -4.64 | 7.58 | -5.85 | 8.03 | -7.03 | 8.39 | -8.17 | 8.68 | -9.26 | 8.88 | -10.30 | 9.01 | -11.27 | 9.05 | -12.17 | 9.00 | -13.00 |
| 7.84 | -5.41 | 8.40 | -6.67 | 8.89 | -7.89 | 9.30 | -9.07 | 9.62 | -10.20 | 9.85 | -11.26 | 9.99 | -12.25 | 10.04 | -13.17 | 10.00 | -14.00 |
| 8.60 | -6.17 | 9.22 | -7.49 | 9.76 | -8.76 | 10.20 | -9.98 | 10.56 | -11.14 | 10.82 | -12.23 | 10.98 | -13.24 | 11.04 | -14.17 | 11.00 | -15.00 |
| 9.37 | -6.94 | 10.04 | -8.31 | 10.62 | -9.62 | 11.11 | -10.88 | 11.50 | -12.08 | 11.78 | -13.19 | 11.96 | -14.22 | 12.03 | -15.16 | 12.00 | -16.00 |
| 10.13 | -7.71 | 10.86 | -9.13 | 11.49 | -10.49 | 12.02 | -11.79 | 12.44 | -13.02 | 12.75 | -14.16 | 12.95 | -15.21 | 13.03 | -16.16 | 13.00 | -17.00 |
| 10.90 | -8.47 | 11.68 | -9.94 | 12.36 | -11.36 | 12.92 | -12.70 | 13.38 | -13.96 | 13.71 | -15.13 | 13.93 | -16.19 | 14.03 | -17.15 | 14.00 | -18.00 |
| 11.67 | -9.24 | 12.50 | -10.76 | 13.22 | -12.22 | 13.83 | -13.60 | 14.32 | -14.90 | 14.68 | -16.09 | 14.92 | -17.18 | 15.02 | -18.15 | 15.00 | -19.00 |
| 12.43 | -10.00 | 13.32 | -11.58 | 14.09 | -13.09 | 14.74 | -14.51 | 15.26 | -15.84 | 15.65 | -17.06 | 15.90 | -18.16 | 16.02 | -19.15 | 16.00 | -20.00 |
| 13.20 | -10.77 | 14.14 | -12.40 | 14.95 | -13.95 | 15.64 | -15.42 | 16.20 | -16.78 | 16.61 | -18.02 | 16.88 | -19.15 | 17.01 | -20.14 | 17.00 | -21.00 |
| 13.96 | -11.54 | 14.96 | -13.22 | 15.82 | -14.82 | 16.55 | -16.32 | 17.14 | -17.72 | 17.58 | -18.99 | 17.87 | -20.13 | 18.01 | -21.14 | 18.00 | -22.00 |

EP 3 766 472 A1

## FIG. 10

EP 3 766 472 A1

## INTERNATIONAL SEARCH REPORT

International application No.

PCT/JP2019/009883

### A. CLASSIFICATION OF SUBJECT MATTER
Int.Cl. A61J1/05(2006.01)i

According to International Patent Classification (IPC) or to both national classification and IPC

### B. FIELDS SEARCHED

Minimum documentation searched (classification system followed by classification symbols)
Int.Cl. A61J1/05

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2002-248157 A (SANTEN PHARMACEUTICAL CO., LTD.) 03 September 2002, paragraphs [0030]-[0056], fig. 3-4, 7-8 (Family: none) | 1-6 |
| Y | JP 2006-20908 A (TAISEI SANGYO KK) 26 January 2006, paragraphs [0016]-[0031], fig. 1-7 (Family: none) | 1-6 |

☒ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 April 2019 (12.04.2019) | 23 April 2019 (23.04.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

23

**INTERNATIONAL SEARCH REPORT**

| International application No. |
|---|
| PCT/JP2019/009883 |

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 3145394 U (TANAKA, Isamu) 09 October 2008, paragraphs [0007]-[0009], fig. 1-3 (Family: none) | 1-6 |
| Y<br>A | US 4733802 A (SHELDON, Gerald M.) 29 March 1988, column 3, line 61 to column 6, line 14, fig. 1-3 (Family: none) | 2<br>1, 3-6 |
| Y<br>A | Microfilm of the specification and drawings annexed to the request of Japanese Utility Model Application No. 144905/1987 (Laid-open No. 062094/1988) (DR. KARL THOMAE GMBH) 26 May 1978, column 2, lines 13-19, fig. 2-7 & FR 2368936 A1 | 3<br>1-2, 5-6 |
| Y<br>A | KR 10-2012-0020589 A (KIM, In-Hwan) 08 March 2012, paragraphs [0020]-[0039], fig. 1-3 (Family: none) | 4<br>1-3, 5-6 |
| A | US 5030214 A (SPECTOR, Larry) 09 July 1991, entire text, all drawings (Family: none) | 1-6 |

Form PCT/ISA/210 (continuation of second sheet) (January 2015)

**EP 3 766 472 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016508054 PCT